# EUROPÄISCHE PATENTSCHRIFT

(11) **EP 0 781 349 B1**
(45) Veröffentlichungstag und Bekanntmachung des Hinweises auf die Patenterteilung: **15.01.2003**
(21) Anmeldenummer: 95932002.9
(22) Anmeldetag: 07.09.1995
(51) Int. Cl.: C12Q 1/04, C12N 9/36, C12N 15/55

(54) **AUFSCHLUSS VON BAKTERIENZELLEN DURCH PHAGENLYSINE**
DECOMPOSITION OF BACTERIA CELLS BY PHAGE LYSINS
DISSOLUTION DE CELLULES BACTERIENNES PAR DES LYSINES DE PHAGES

(30) Priorität: 09.09.1994 DE 4432053; 24.02.1995 DE 19506615; 06.03.1995 EP 95301416
(43) Veröffentlichungstag der Anmeldung: 02.07.1997
(73) Patentinhaber: Biotec Laboratories Limited, Ipswich, Suffolk IP5 7RG (GB)
(72) Erfinder: SCHERER, Siegfried, D-85350 Freising (DE); LÖSSNER, Martin, D-85350 Freising (DE); STEWART, Gordon, S., A., B., Loughborough, Leicestershire L11 0QL (GB); SCHUBERT, Peter, D-64285 Darmstadt (DE)
(74) Vertreter: Wilkinson, Stephen John
(86) Internationale Anmeldenummer: EP9503512
(87) Internationale Veröffentlichungsnummer: WO96007756

(56) Entgegenhaltungen:
- EP-A- 0 510 907
- WO-A-94/06931
- WO-A-95/05483
- NATO ASI SERIES, Bd. H65, 1992 Seiten 427-431, J. PAYNE ET AL. 'Cloning and characterisation of a lysin gene from Listeria bacteriophage.' in der Anmeldung erwähnt
- APPLIED AND ENVIRONMENTAL MICROBIOLOGY, Bd. 58, Nr. 1, - Januar 1992 Seiten 296-302, R. ZINK ET AL. 'Classification of virulent and temperate bacteriophages of Listeria spp. on the basis of morphology and protein analysis.'
- APPLIED AND ENVIRONMENTAL MICROBIOLOGY, Bd. 61, Nr. 3, März 1995 Seiten 1150-1152, M. J. LOOESSNER ET AL. 'A new procedure for efficient recovery of DNA, RNA, and proteins from Listeria cells by rapid lysis with a recombinant bacteriophage endolysin.'
- MOLECULAR MICROBIOLOGY, Bd. 16, Nr. 6, 26.Juli 1995 Seiten 1231-1241, M. J. LOESANER ETAL. 'Heterogeneous endolysins in Listeria monocytogenes bacteriophages: a new class of enzymes and evidence for conserved holin genes within the siphoviral lysis cassettes.'

### SEQUENZPROTOKOLL

### (1) ALLGEMEINE ANGABEN:

(i) ANMELDER:
   (A) NAME: MERCK PATENT GmbH
   (B) STRASSE: Frankfurter Str.
   (C) ORT: Darmstadt
   (E) LAND: Germany
   (F) POSTLEITZAHL: 64293
   (G) TELEFON: (06151) 72 78 40
   (H) TELEFAX: (06151) 72 71 91
(ii) BEZEICHNUNG DER ERFINDUNG: Aufschluss von Bakterienzellen durch Phagenlysine
(iii) ANZAHL DER SEQUENZEN: 6
(iv) COMPUTER-LESBARE FASSUNG:
   (A) DATENTRÄGER: Floppy disk
   (B) COMPUTER: IBM PC compatible
   (C) BETRIEBSSYSTEM: PC-DOS/MS-DOS
   (D) SOFTWARE: PatentIn Release #1.0, Version #1.30 (EPA)
(vi) DATEN DER URANMELDUNG:
   (A) ANMELDENUMMER: DE 44 32 053.1
   (B) ANMELDETAG: 09-SEP-1994
(vi) DATEN DER URANMELDUNG:
   (A) ANMELDENUMMER: DE 195 06 615.4
   (B) ANMELDETAG: 23-FEB-1995
(vi) DATEN DER URANMELDUNG:
   (A) ANMELDENUMMER: EP 95 301 416.4
   (B) ANMELDETAG: 06-MAR-1995

### (2) ANGABEN ZU SEQ ID NO: 1:

(i) SEQUENZKENNZEICHEN:
   (A) LANGE: 846 Basenpaare
   (B) ART: Nucleotid
   (C) STRANGFORM: nicht bekannt
   (D) TOPOLOGIE: nicht bekannt
(ii) ART DES MOLEKÜLS: Genom-DNA
(vi) URSPRÜNLICHE HERKUNFT:
   (B) STAMM: A 118 Listeria - Phage
(xi) SEQUENZBESCHREIBUNG: SEQ ID NO: 1:
   ATGACAAGTT ATTATTATAG TAGAAGTTTA GCGAATGTAA ATAAGTTAGC AGACAATACG   60
   AAAGCGGCAC GTAGAAAATT GCTAGATTGG TCTGAAAGCA ACGGGATTGA AGTATTAATC  120
   TACGAAACAA TTAGAACGAA AGAACAACAA GCCGCAAATG TTAACAGTGG AGCGTCTCAA  180
   ACAATGCGCT CTTATCACTT GGTAGGACAA GCGCTAGATT TCGTCATGGC GAAAGGTAAA  240
   ACGGTCGATT GGGGTGCTTA TCGTTCAGAC AAAGGCAAGA AATTTGTGGC AAAGGCAAAA  300
   TCTTTAGGTT TTGAGTGGGG TGGTGATTGG TCTGGATTTG TAGACAATCC GCACCTTCAA  360
   TTTAATTATA AAGGCTATGG GACTGATACT TTTGGAAAAG GAGCTAGTAC TAGTAATTCA  420
   TCTAAACCGA GCGCAGACAC AAACACAAAC AGTCTAGGAT TAGTAGATTA TATGAATTTA  480
   AATAAACTAG ATTCAAGCTT TGCGAATCGC AAAAAACTAG CGACAAGTTA CGGAATTAAA  540
   AATTACAGTG GAACAGCAAC GCAGAACACA ACATTATTAG CGAAGTTAAA AGCAGGAAAA  600
   CCACACACAC CAGCAAGCAA AAACACATAC TACACAGAAA ATCCGCGAAA AGTTAAAACA  660
   CTAGTACAAT GTGATCTATA CAAATCAGTA GACTTTACAA CAAAAAACCA AACAGGTGGA  720
   ACATTTCCGC CAGGCACAGT CTTCACGATT TCAGGGATGG GGAAAACGAA AGGCGGAACA  780
   CCTCGCTTGA AGACGAAGAG CGGTTACTAT CTCACTGCTA ACACGAAGTT TGTTAAAAAG  840
   ATTTAG                                                             846

### (2) ANGABEN ZU SEQ ID NO: 2:

(i) SEQUENZKENNZEICHEN:
   (A) LÄNGE: 870 Basenpaare
   (B) ART: Nucleotid
   (C) STRANGFORM: nicht bekannt
   (D) TOPOLOGIE: nicht bekannt
(ii) ART DES MOLEKÜLS: Genom-DNA
(vi) URSPRÜNLICHE HERKUNFT:
   (B) STAMM: A 500 Listeria - Phage
(xi) SEQUENZBESCHREIBUNG: SEQ ID NO: 2:
   ATGGCATTAA CAGAGGCATG GCTAATTGAA AAAGCAAATC GCAAATTGAA TGCTGGGGGA    60
   ATGTATAAAA TTACATCGGA TAAAACACGA AATGTAATTA AAAAAATGGC AAAAGAAGGT   120
   ATTTATCTTT GTGTTGCGCA AGGTTACCGC TCAACAGCGG AACAAAATGC GCTATATGCA   180
   CAAGGGAGAA CCAAACCTGG AGCAATTGTT ACTAATGCCA AGGGCGGGCA ATCTAATCAC   240
   AACTACGGGG TAGCTGTTGA CTTGTGCTTG TATACAAATG ACGGAAAAGA TGTTATTTGG   300
   GAGTCAACAA CTTCCCGGTG GAAAAAGGTT GTTGCTGCTA TGAAAGCAGA AGGGTTTAAA   360
   TGGGGCGGAG ACTGGAAAAG TTTTAAAGAC TATCCGCATT TTGAACTATG TGATGCTGTA   420
   AGTGGTGAGA AAATCCCTGC TGCAACACAA AACACTAATA CAAATTCAAA TCGTTACGAG   480
   GGTAAAGTCA TTGATAGCGC ACCACTGCTA CCGAAAATGG ACTTTAAATC ATCACCATTC   540
   CGCATGTATA AGGTAGGAAC TGAGTTCTTA GTATATGATC ATAATCAATA TTGGTACAAG   600
   ACATACATTG ATGACAAACT TTACTACATG TATAAAAGCT TTTGCGATGT TGTAGCTAAA   660
   AAAGACGCAA AAGGTCGCAT CAAAGTTCGA ATTAAAAGCG CGAAAGACTT GCGTATTCCA   720
   GTCTGGAATA ACATAAAATT GAATTCTGGG AAAATTAAAT GGTATGCACC CAATGTAAAA   780
   CTAGCGTGGT ACAACTATCG AAGAGGATAT TTAGAGCTAT GGTATCCGAA CGACGGCTGG   840
   TATTACACAG CAGAATACTT CTTAAAATAA                                    870

### (2) ANGABEN ZU SEQ ID NO: 3:

(i) SEQUENZKENNZEICHEN:
   (A) LÄNGE: 1026 Basenpaare
   (B) ART: Nucleotid
   (C) STRANGFORM: nicht bekannt
   (D) TOPOLOGIE: nicht bekannt
(ii) ART DES MOLEKÜLS: Genom-DNA
(vi) URSPRÜNLICHE HERKUNFT:
   (B) STAMM: A 511 Listeria - Phage
(xi) SEQUENZBESCHREIBUNG: SEQ ID NO: 3:
   ATGGTAAAAT ATACCGTAGA GAACAAAATT ATTGCAGGAT TACCTAAAGG TAAACTAAAA     60
   GGGGCTAACT TTGTTATTGC TCATGAAACT GCAAATAGCA AGTCTACTAT TGACAATGAA    120
   GTAAGCTACA TGACTAGGAA CTGGAAGAAC GCATTTGTAA CTCACTTTGT AGGTGGCGGA    180
   GGTAGAGTCG TTCAGGTTGC TAATGTAAAC TATGTTTCTT GGGGAGCAGG TCAGTATGCT    240
   AACTCTTATT CCTATGCGCA GGTAGAGTTG TGCCGTACAA GTAATGCAAC TACATTTAAG    300
   AAAGACTATG AAGTGTACTG TCAATTACTA GTAGACCTAG CTAAAAAAGC AGGTATCCCT    360
   ATTACACTTG ACTCTGGTAG TAAAACTAGT GATAAAGGTA TTAAATCCCA TAAATGGGTT    420
   GCTGATAAGC TAGGAGGAAC AACACACCAA GACCCATATG CTTACTTAAG CTCATGGGGT    480
   ATTAGTAAAG CACAATTTGC TAGTGACTTG GCTAAAGTAT CTGGCGGAGG AAACACAGGA    540
   ACAGCGCCAG CTAAACCAAG CACACCAGCA CCTAAACCAA GCACACCATC TACTAACCTA    600
   GACAAACTTG GCTTAGTAGA CTACATGAAC GCTAAGAAAA TGGACTCTAG CTACAGTAAC    660
   AGAGATAAGT TAGCTAAACA GTATGGTATT GCTAACTATT CAGGAACAGC TAGCCAGAAC    720
   ACTACACTCC TTAGTAAAAT TAAAGGAGGA GCACCTAAAC CAAGCACACC AGCACCTAAA    780
   CCTAGTACAT CTACAGCTAA GAAAATTTAT TTCCCACCAA ATAAAGGAAA CTGGTCTGTG    840
   TATCCAACAA ATAAAGCACC CGTTAAGGCT AATGCTATTG GTGCTATTAA CCCTACTAAA    900
   TTCGGAGGAT TGACTTACAC TATCCAAAAA GATAGAGGAA ACGGTGTATA CGAAATCCAA    960
   ACAGACCAAT TCGGCAGAGT TCAAGTCTAT GGTGCACCTA GTACAGGAGC AGTTATCAAA   1020
   AAATAA                                                              1026

### (2) ANGABEN ZU SEQ ID NO: 4:

(i) SEQUENZKENNZEICHEN:
   (A) LÄNGE: 281 Aminosäuren
   (B) ART: Aminosäure
   (C) STRANGFORM: Einzelstrang
   (D) TOPOLOGIE: linear
(ii) ART DES MOLEKÜLS: Protein
(vi) URSPRÜNLICHE HERKUNFT:
   (B) STAMM: A118 Listeria - Phage
(xi) SEQUENZBESCHREIBUNG: SEQ ID NO: 4:
   Met Thr Ser Tyr Tyr Tyr Ser Arg Ser Leu Ala Asn Val Asn Lys Leu
   1               5                   10                  15
   Ala Asp Asn Thr Lys Ala Ala Arg Arg Lys Leu Leu Asp Trp Ser Glu
               20                  25                  30
   Ser Asn Gly Ile Glu Val Leu Ile Tyr Glu Thr Ile Arg Thr Lys Glu
           35                  40                  45
   Gln Gln Ala Ala Asn Val Asn Ser Gly Ala Ser Gln Thr Met Arg Ser
       50                  55                  60
   Tyr His Leu Val Gly Gln Ala Leu Asp Phe Val Met Ala Lys Gly Lys
   65                  70                  75                  80
   Thr Val Asp Trp Gly Ala Tyr Arg Ser Asp Lys Gly Lys Lys Phe Val
                   85                  90                  95
   Ala Lys Ala Lys Ser Leu Gly Phe Glu Trp Gly Gly Asp Trp Ser Gly
               100                 105                 110
   Phe Val Asp Asn Pro His Leu Gln Phe Asn Tyr Lys Gly Tyr Gly Thr
           115                 120                 125
   Asp Thr Phe Gly Lys Gly Ala Ser Thr Ser Asn Ser Ser Lys Pro Ser
       130                 135                 140
   Ala Asp Thr Asn Thr Asn Ser Leu Gly Leu Val Asp Tyr Met Asn Leu
   145                 150                 155                 160
   Asn Lys Leu Asp Ser Ser Phe Ala Asn Arg Lys Lys Leu Ala Thr Ser
                   165                 170                 175
   Tyr Gly Ile Lys Asn Tyr Ser Gly Thr Ala Thr Gln Asn Thr Thr Leu
               180                 185                 190
   Leu Ala Lys Leu Lys Ala Gly Lys Pro His Thr Pro Ala Ser Lys Asn
           195                 200                 205
   Thr Tyr Tyr Thr Glu Asn Pro Arg Lys Val Lys Thr Leu Val Gln Cys
       210                 215                 220
   Asp Leu Tyr Lys Ser Val Asp Phe Thr Thr Lys Asn Gln Thr Gly Gly
   225                 230                 235                 240
   Thr Phe Pro Pro Gly Thr Val Phe Thr Ile Ser Gly Met Gly Lys Thr
                   245                 250                 255
   Lys Gly Gly Thr Pro Arg Leu Lys Thr Lys Ser Gly Tyr Tyr Leu Thr
               260                 265                 270
   Ala Asn Thr Lys Phe Val Lys Lys Ile
           275                 280

### (2) ANGABEN ZU SEQ ID NO: 5:

(i) SEQUENZKENNZEICHEN:
   (A) LÄNGE: 289 Aminosäuren
   (B) ART: Aminosäure
   (C) STRANGFORM: Einzelstrang
   (D) TOPOLOGIE: linear
(ii) ART DES MOLEKÜLS: Protein
(vi) URSPRÜNLICHE HERKUNFT:
   (B) STAMM: A 500 Listeria - Phage
(xi) SEQUENZBESCHREIBUNG: SEQ ID NO: 5:
   Met Ala Leu Thr Glu Ala Trp Leu Ile Glu Lys Ala Asn Arg Lys Leu
   1               5                   10                  15
   Asn Ala Gly Gly Met Tyr Lys Ile Thr Ser Asp Lys Thr Arg Asn Val
               20                  25                  30
   Ile Lys Lys Met Ala Lys Glu Gly Ile Tyr Leu Cys Val Ala Gln Gly
           35                  40                  45
   Tyr Arg Ser Thr Ala Glu Gln Asn Ala Leu Tyr Ala Gln Gly Arg Thr
       50                  55                  60
   Lys Pro Gly Ala Ile Val Thr Asn Ala Lys Gly Gly Gln Ser Asn His
   65                  70                  75                  80
   Asn Tyr Gly Val Ala Val Asp Leu Cys Leu Tyr Thr Asn Asp Gly Lys
                   85                  90                  95
   Asp Val Ile Trp Glu Ser Thr Thr Ser Arg Trp Lys Lys Val Val Ala
               100                 105                 110
   Ala Met Lys Ala Glu Gly Phe Lys Trp Gly Gly Asp Trp Lys Ser Phe
           115                 120                 125
   Lys Asp Tyr Pro His Phe Glu Leu Cys Asp Ala Val Ser Gly Glu Lys
       130                 135                 140
   Ile Pro Ala Ala Thr Gln Asn Thr Asn Thr Asn Ser Asn Arg Tyr Glu
   145                 150                 155                 160
   Gly Lys Val Ile Asp Ser Ala Pro Leu Leu Pro Lys Met Asp Phe Lys
                   165                 170                 175
   Ser Ser Pro Phe Arg Met Tyr Lys Val Gly Thr Glu Phe Leu Val Tyr
               180                 185                 190
   Asp His Asn Gln Tyr Trp Tyr Lys Thr Tyr Ile Asp Asp Lys Leu Tyr
           195                 200                 205
   Tyr Met Tyr Lys Ser Phe Cys Asp Val Val Ala Lys Lys Asp Ala Lys
       210                 215                 220
   Gly Arg Ile Lys Val Arg Ile Lys Ser Ala Lys Asp Leu Arg Ile Pro
   225                 230                 235                 240
   Val Trp Asn Asn Ile Lys Leu Asn Ser Gly Lys Ile Lys Trp Tyr Ala
                   245                 250                 255
   Pro Asn Val Lys Leu Ala Trp Tyr Asn Tyr Arg Arg Gly Tyr Leu Glu
               260                 265                 270
   Leu Trp Tyr Pro Asn Asp Gly Trp Tyr Tyr Thr Ala Glu Tyr Phe Leu
           275                 280                 285
   Lys

### (2) ANGABEN ZU SEQ ID NO: 6:

(i) SEQUENZKENNZEICHEN:
   (A) LÄNGE: 341 Aminosäuren
   (B) ART: Aminosäure
   (C) STRANGFORM: Einzelstrang
   (D) TOPOLOGIE: linear
(ii) ART DES MOLEKÜLS: Protein
(vi) URSPRÜNLICHE HERKUNFT:
   (B) STAMM: A 511 Listeria - Phage
(xi) SEQUENZBESCHREIBUNG: SEQ ID NO: 6:
   Met Val Lys Tyr Thr Val Glu Asn Lys Ile Ile Ala Gly Leu Pro Lys
   1               5                   10                  15
   Gly Lys Leu Lys Gly Ala Asn Phe Val Ile Ala His Glu Thr Ala Asn
               20                  25                  30
   Ser Lys Ser Thr Ile Asp Asn Glu Val Ser Tyr Met Thr Arg Asn Trp
           35                  40                  45
   Lys Asn Ala Phe Val Thr His Phe Val Gly Gly Gly Gly Arg Val Val
       50                  55                  60
   Gln Val Ala Asn Val Asn Tyr Val Ser Trp Gly Ala Gly Gln Tyr Ala
   65                  70                  75                  80
   Asn Ser Tyr Ser Tyr Ala Gln Val Glu Leu Cys Arg Thr Ser Asn Ala
                   85                  90                  95
   Thr Thr Phe Lys Lys Asp Tyr Glu Val Tyr Cys Gln Leu Leu Val Asp
               100                 105                 110
   Leu Ala Lys Lys Ala Gly Ile Pro Ile Thr Leu Asp Ser Gly Ser Lys
           115                 120                 125
   Thr Ser Asp Lys Gly Ile Lys Ser His Lys Trp Val Ala Asp Lys Leu
       130                 135                 140
   Gly Gly Thr Thr His Gln Asp Pro Tyr Ala Tyr Leu Ser Ser Trp Gly
   145                 150                 155                 160
   Ile Ser Lys Ala Gln Phe Ala Ser Asp Leu Ala Lys Val Ser Gly Gly
                   165                 170                 175
   Gly Asn Thr Gly Thr Ala Pro Ala Lys Pro Ser Thr Pro Ala Pro Lys
               180                 185                 190
   Pro Ser Thr Pro Ser Thr Asn Leu Asp Lys Leu Gly Leu Val Asp Tyr
           195                 200                 205
   Met Asn Ala Lys Lys Met Asp Ser Ser Tyr Ser Asn Arg Asp Lys Leu
       210                 215                 220
   Ala Lys Gln Tyr Gly Ile Ala Asn Tyr Ser Gly Thr Ala Ser Gln Asn
   225                 230                 235                 240
   Thr Thr Leu Leu Ser Lys Ile Lys Gly Gly Ala Pro Lys Pro Ser Thr
                   245                 250                 255
   Pro Ala Pro Lys Pro Ser Thr Ser Thr Ala Lys Lys Ile Tyr Phe Pro
               260                 265                 270
   Pro Asn Lys Gly Asn Trp Ser Val Tyr Pro Thr Asn Lys Ala Pro Val
           275                 280                 285
   Lys Ala Asn Ala Ile Gly Ala Ile Asn Pro Thr Lys Phe Gly Gly Leu
       290                 295                 300
   Thr Tyr Thr Ile Gln Lys Asp Arg Gly Asn Gly Val Tyr Glu Ile Gln
   305                 310                 315                 320
   Thr Asp Gln Phe Gly Arg Val Gln Val Tyr Gly Ala Pro Ser Thr Gly
                   325                 330                 335
   Ala Val Ile Lys Lys
               340

## Beschreibung

### Gebiet der Erfindung

Die Erfindung betrifft bestimmte Phagenlysine und Nukleinsäuresequenzen, die diese Phagenlysine codieren.

### Stand der Technik

Für den Nachweis und die Bestimmung von Bakterien dienen neben extrazellulären Bestandteilen oft zelluläre Bestandteile, z.B. Adenosintriphosphat (ATP) für den Nachweis von Bakterien oder chromosomale DNS oder ribosomale RNS als Zielanalyt für Nukleinsäure-Sonden oder für die Nukleinsäureamplifikation. Zur Untersuchung derartiger zellulären Bestandteile von Bakterien (z.B. chromosomale DNS, Plasmide, RNS, Proteine, Lipide, Metabolite) müssen die Zellen möglichst schnell und schonend aufgeschlossen werden, um die Integrität und Funktionalität der isolierten Bestandteile und Makromoleküle zu gewährleisten und um die Analyte zur Steigerung der Nachweisempfindlichkeit in hoher Ausbeute zu isolieren. Zu den bekannten Methoden gehört die Anwendung von Lysozym oder Mutanolysin (lysierendes Enzym aus Streptomyces globisporus). Auch in Kombination mit Detergenzien erfordert diese Methode erhöhte Temperaturen und ein alkalisches Medium bei oft langen Inkubationszeiten. Unter diesen Bedingungen sind viele Analyte instabil.

Für den Nachweis von Bakterien wird in WO 94/06 931 vorgeschlagen, die Bakterienzellen mit Phagen zu lysieren und anschließend beispielsweise das ATP mittels bekannter Methoden nachzuweisen. Bakteriophagen weisen im allgemeinen enge Wirtsbereiche auf. Bei der Durchführung dieses Verfahrens muß außerdem mit vermehrungsfähigen Viren gearbeitet werden; eine Verfahrenweise, die für die Routineanalytik aus Sicherheitsgründen bedenklich ist. In EP 0 168 933 werden rekombinante Phagen offenbart, die das lux-Gen insertiert enthatten.

Phagenlysine aus verschiedenen phageninfizierten Bakterien wurden beschrieben. In DE 43 26 617 wird beispielsweise die Verwendung von Lysinen aus Listeriaphagen als spezifisches Desinfektionsmittel gegen Listerien offenbart. Weitere Listeriaphagen sind in der Literatur beschrieben: z.B. von Loessner und Busse (1990) Appl.Environ.Microbiol. **56,** Seiten 1912 - 1918, und von Loessner et al. (1994) J.Gen.Virol. **75,** Seiten 701 - 710. Dabei wurde gefunden, daß bestimmte Phagenlysine Zellen von Bakterien der Gattung Listeria lysieren, wohingegen der Phage, durch dessen genetische Information diese Lysine codiert werden, lediglich bestimmte Listeria-Arten oder sogar nur bestimmte Listeria-Stämme infiziert und lysiert. Durch Phagenlysine hervorgerufenen Bakteriolyse kann äußerst spezifisch sein; einige Bakteriophagen infizieren und lysieren nur bestimmte Serotypen von Bakterien. Es gibt aber auch Phagenlysine mit wesentich breiterem Lysespektrum: So wird beispielsweise in JP 04/141 089 ein Phagolysin offenbart, das ein ähnlich breites Lysespektrum wie Lysozym aufweist. Eigenschaften von Phagenlysinen aus Bacteriophagen, die Gram-negative Bakterien infizieren, wurden von Young (1992) in Microbiol. Rev. **56**, Seiten 430 - 481 zusammengestellt. Lysine aus Phagen, die Gram-positive Bakterien infizieren, sind von verschiedenen Autoren beschrieben: Paces et al. (1986) Gene **44,** Seiten 115 - 120; Saedi et al. (1987) Proc. Natl. Acad. Sci. USA **84,** Seiten 955 - 958; Boizet et al. (1990) Gene **94,** Seiten 61 - 67; Garcia et al. (1990) Gene **86,** Seiten 137 - 142; Ward et al. (1993) Canad. J. Microbiol. **39,** Seiten 767 - 774; Shearman et al. (1994) Mol. Gen. Genet. **216,** Seiten 214 - 221. In J. Bact. **107,** Seite 499 - 504 (1971) werden Phagenlysine beschrieben, die spezifisch Zellen der Bakteriengattung Staphylococcus lysieren. In Chemical Abstracts **84** 1596 (1976) wird auf Phagenlysine hingewiesen, die nur bestimmte Clostridienarten lysieren.

Gegenstand der Erfindung sind gereinigte Lysine aus Listeriaphagen der Stämme A118, A500 und A511, sowie die Nukleinsäuresequenzen, die die Lysine codieren.

### Beschreibung der Erfindung

Nachweisverfahren für Bakterien, bei denen zelluläre bakterielle Bestandteile zur Reaktion gebracht werden, sind in großer Zahl bekannt. Es war auch bekannt, dass unter zellulären Bestandteilen neben Bestandteilen aus dem Zellinnern auch durch die Lyse freigesetzte Bestandteile der Zellwand verstanden werden. Dazu gehören der Nachweis von Enzymen, die nur bei bestimmten Species oder Gruppen von Bakterien vorkommen, z.B. Glucosidase, β-Galactosidase oder Enzyme der oxydativen Phosphorylierung (siehe z.B. EP-A-0 510 907). Weiterhin gehören dazu Nachweisreaktionen auf Nukleinsäuren mittels Nukleinsäuresonden oder Amplifikationsverfahren. Ein weiteres bekanntes Nachweisverfahren beruht auf dem Nachweis von ATP, beispielsweise durch die Messung der Lumineszenz, die bei der Luciferin-Luciferase-Reaktion auftritt. Derartige Verfahren, sowie die Optimierung von Verfahrensvarianten sind dem Fachmann geläufig und in der Literatur beschrieben. Erfindungsgemäß werden diese Reaktionen als übliche Nachweisverfahren zusammengefaßt. Die Reagenzien, die für diese Nachweisverfahren notwendig sind, werden als übliche Nachweismittel bezeichnet.

Beispiele für derartige Nachweismittel sind folgende Zusammenstellungen:
a) für den Nachweis von Enzymen umfassend einen Puffer und ein Enzymsubstrat ;
b) für den Nachweis spezieller Nukleinsäureabschnitte umfassend eine Nukleinsäuresonde, die mittels einer Markierung nachweisbar ist;
c) für die Amplifikation spezieller Nukleinsäureabschnitte umfassend zwei Nukleinsäureprimer, ein Gemisch der Nukleotidtriphosphate und eine Nukleinsäurepolymerase;
d) für den Nachweis von ATP umfassend Luciferin und Luciferase beispielsweise aus Leuchtkäfern.

So werden in Infect.Immunity **58**, 1943 - 1950 (1990) für den Nachweis von Listerien geeigntet DNS-Sonden beschrieben. Für den Nachweis von Listerien geeignete Primer werden in EP 0 576 842 offenbart. Beide Dokumente offenbaren Einzelheiten über Verfahrensparameter für derartige Nachweisreaktionen, sowie weitere Literatur, die zugehörige Verfahrensvarianten offenbart.

Der ATP-Nachweis mit der Luciferin-Luciferase-Reaktion ist nicht spezifisch für bestimmte Organismen, da ATP in allen lebenden Zellen vorkommt. Wird dieser Nachweis jedoch erfindungsgemäß mit einem Phagenlysin kombiniert, das spezifisch für eine bestimmte Bakteriengattung ist, so wird die Nachweisreaktion insgesamt spezifisch. Wird mittels eines Phagenlysins mit breitem Wirkungsspektrum DNA freigesetzt und erfolgt der Nachweis mittels einer spezifischen DNA-Sonde, so ist wiederum die Nachweisreaktion insgesamt spezifisch.

Phagenlysine sind Enzyme oder Enzymkomplexe, die Lysis von Bakterien bewirken, und die im Phagengenom codiert sind. Phagenlysine wurden aus zahlreichen mit Bakteriophagen infizierten Bakterienzellen gewonnen und aufgereinigt. Entsprechend ihrer Spaltungsspezifität gehören Phagenlysine zu drei verschiedenen Enzymgruppen: Glucosidasen, Amidasen und Endopeptidasen. Beispiele für solche dem Fachmann bekannten Phagenlysine sind in den bereits genannten Dokumenten offenbart. Phagenlysine können aus kultivierten phageninfizierten Bakterien gewonnen werden. Bevorzugterweise werden die Phagenlysine jedoch aus Kulturen von Bakterien, z.B. Escherichia coli, gewonnen, wobei die Bakterien durch dem Fachmann bekannte Rekombinationstechniken in die Lage versetzt werden, die gewünschten Phagenlysine zu produzieren, siehe z.B. EP-A-0 510 907.

Aus Lysaten von Bakterien der Gattung Listeria, die mit Listeriaphagen A 511, A 500 oder A 118 infiziert wurden, können Phagenllysine (bezeichnet als PLY 511, PLY 500 beziehungsweise PLY 118) hergestellt werden, die eine schnelle (d.h. innerhalb von 2-6 Minuten) und vollständige (95-100 %) Zellwand-Hydrolyse ermöglicht. Dabei werden neben Bestandteilen des Zellinnern (chromosomale DNS), Plasmide, RNS, Proteine, etc.) auch sämtliche Zellwand-assoziierten (nicht-kovalent gebundenen) Proteine freigesetzt. Alle Makromoleküle liegen anschließend nativ vor, sind aktiv und keiner chemischen Schädigung ausgesetzt. Erfindungsgemäß verwendbare Phagenlysine können auch durch Rekombinationstechniken, beispielsweise aus rekombinanten Stämmen von Escherichia coli, gewonnen werden, die die Phagolysin-Gene *ply* 511, *ply* 500 oder *ply* 118 (aus den Phagen A 511, A 500 beziehungsweise A 118) auf einem Expressionsplasmid tragen. Hierbei können die Zellen in hoher Ausbeute (bis zu 20 % des Gesamt-Proteins) Lysine produzieren. Diese rekombinanten Enzyme lassen sich leichter isolieren und reinigen, und weisen dieselbe Spezifität bei keiner nachweisbaren Fremdaktivität auf. Die gereinigten Phagenlysine werden in einem wäßrigen Puffer bei -30 °C aufbewahrt. Die Phagenlysine PLY 511, PLY 500 und PLY 118, sowie die zugehörigen Gene *ply* 511, *ply* 500 und *ply* 118 wurden bisher nicht in Einzelheiten beschrieben.

Im ersten Verfahrensschritt wird die Probe, die die nachzuweisenden Bakterien enthält, mit einem Phagenlysin unter Bedingungen, die die Lyse erlauben, inkubiert. Entsprechend der analytischen Aufgabe wird dabei ein Lysin mit geeigneter Spezifität eingesetzt, z.B. für den Nachweis von Listerien das Phagenlysin PLY118. Für derartige enzymatische Reaktionen geeignete und übliche Bedingungen sind grundsätzlich dem Fachmann bekannt: Die Inkubationstemperatur beträgt typischerweise 4 - 40 °C oder auch Raumtemperatur (20 - 30 °C); die Inkubationsdauer beträgt 2 - 20 Minuten in Abhängigkeit von der eingesetzten Enzymmenge; die Reaktion wird bei einem pH von 5 - 10, insbesondere von 7 - 9, ausgeführt. Die freigesetzten zellulären Bestandteile der Bakterien werden anschließend wie bereits erwähnt nachgewiesen.

Erfindungsgemäß werden unter dem Begriff gereinigte Phagenlysine Enzympräparationen verstanden, die durch dem Fachmann grundsätzlich bekannten Verfahrensschritten aus einem Rohextrakt soweit gereinigt wurden, daß sie für die Lyse geeignet sind und beispielsweise die folgende Nachweisreaktion störendes Material, beispielsweise aus dem Rohextrakt stammendes ATP oder aus dem Rohextrakt stammende Nukleinsäuren, entfernt sind.

Es ist dem Fachmann bekannt, daß häufig wesentliche Eigenschaften von Proteinen durch den Austausch von wenigen Aminosäuren nicht verändert werden. Dies gilt insbesondere, wenn die ausgetauschten Aminosäuren ähnliche Eigenschaften aufweisen. Zusammenstellungen von Gruppen von Aminosäuren mit ähnlichen Eigenschaften sind beispielsweise von M.O. Dayhoff (1972) Atlas of Protein Sequence and Structure Vol. 5, Seite 98 beschrieben. Derartig abgewandelte Proteine werden häufig als Muteine bezeichnet. Deswegen umfassen die in dieser Anmeldung mit ihrer Aminosäuresequenz offenbarten Lysine aus Listeriaphagen (PLY511, PLY500 und PLY118) neben den in den SEQ.ID.NO.'s: 4 - 6 angegebenen Sequenzen auch zugehörige Muteine, insbesondere solche, die durch Aminosäureaustausch entsprechend den Tabellen von M.O. Dayhoff aus der offenbarten Sequenz hervorgehen.

Dem Fachmann ist weiterhin bekannt, daß der genetische Code degeneriert ist: Bestimmte Aminosäuren in Proteinen können auf verschiedene Weise durch Tripletts von Nukleotiden codiert werden. Soweit erfindungsgemäß Nukleinsäuresequenzen offenbart werden, die bestimmte Proteine codieren, umfaßt diese Offenbarung auch Abwandlungen entsprechend der Degeneration des genetischen Codes.

### Beispiele

Die folgenden Beispiele sollen den Erfindungsgedanken näher erläutern; sie stellen keine Einschränkung des Erfindungsumfanges dar.

### Beispiel 1: Lyse von Bakterienzellen

Zellen von Listeria monocytogenes (WSLC 1001; serovar 1/2c) werden in 10 ml Tryptose-Nährlösung bei 30 °C bis in die spät-logarithmische Wachstumsphase kultiviert und in 2 ml Portionen durch Zentrifugation bei 15 000 x g (1 Minute) geerntet. Für den Lysetest wird der Zellniederschlag in 900 µl Puffer (20 mM Tris-HCI, pH 8,0) resuspendiert, auf 30 °C temperiert und mit 100 µl einer Lösung von 10 U PLY 118 versetzt. (Eine Einheit (U) Phagenlysin verringert in einem Volumen von 1 ml bei einer Temperatur von 30 °C bei einem pH von 8,0 die Extinktion bei 600 nm einer Suspension von Zellen von Listeria monocytogenes WSLC 1001 um 0,01/min.) Die Lysereaktion wird durch Messung der Trübung bei 600 nm verfolgt. Nach 8 - 10 Minuten ist die Lösung klar.

Mit ähnlichem Ergebnis läßt sich der Versuch mit Zellen von Listeria ivanovii (WSLC 3009; serovar 5) und anderen Listeria-Arten wiederholen.

### Beispiel 2: Klonierung, Sequenzermittlung und Analyse der ply-Gene der Listeriaphagen A118, A500 und A511 und deren Genprodukte

Genombibliotheken aus DNA der Listeriaphagen A118, A500 und A511 wurden erstellt, indem DNA-Bruchstücke mit *Taq*l- oder *Tsp*509I-DNasen erzeugt und in *Cla*l- oder *Eco*RI-Positionen von pSP72 integriert wurden. Phagolysin exprimierende Klone wurden durch Nachweis des lysierenden Phänotyps selektiert.

Die insertierten Anteile von positiv reagierenden Klonen wurden durch Behandlung mit Restriktionsendonukleasen aus der Plasmid-DNA gewonnen und durch Southem Hybridisierung mit markierter Phagen-DNA bezüglich Größe und Identität des Fragments untersucht. Von jeweils einem Klon aus jedem Phagen-DNA-Fragment wurde die vollständige Sequenz ermittelt.

In allen drei Fällen war die Zuordnung zu dem lysin-codierenden Leseraster möglich:

| Phage | Gen | | Lysin | | |
|---|---|---|---|---|---|
| | Bezeichnung | Länge | Bezeichnung | Länge | kDalton |
| A118 | *ply*118 | 846 | PLY118 | 281 | 30,8 |
| A500 | *ply*500 | 870 | PLY500 | 289 | 33,4 |
| A511 | *ply*511 | 1024 | PLY511 | 341 | 36,5 |

Die genannten Lysine wurden jeweils aus den E.coli Rohextrakten mittels lonenaustausch- und Gelpermeationschromatographie bis zu einer Homogenität von mindestens 80 % aufgereinigt (SDS-Polyacrylamidgelelektrophorese).

Das pH-Optimum dieser Lysine liegt bei 8,0-8,5; sie sind im pH-Bereich von 5,5 bis 10 aktiv. Viele übliche Puffersysteme können verwendet werden. Die höchste enzymatische Aktivität wurde bei 30 - 37 °C gemessen, jedoch sind die Enzyme auch bei 4 °C aktiv. Natrium- und Kaliumionen aktivieren diese Lysine, ebenso das Detergenz Polyethylenglycolmono-[p-(1,1,3,3-tetramethylbutyl)-phenyl-ether (Triton® X-100; 0,05-0,1 Gew.-%). Dithiothreit im Bereich von 0,1 bis 1,0 mM hatte keinen Effekt auf die Aktivität.

### Beispiel 3: Isolierung von m-RNS

Zur Isolierung von m-RNS aus Kulturen von Listeria monocytogenes werden die Zellen in kleinen Volumina (ca. 1-5 ml) durch Zentrifugation konzentriert, kurz in einem Trockeneis-Ethanol Gemisch eingefroren (ca. 1-5 min.), und in 1/10 Volumen Puffer (Tris-HCl, 20 mM, pH 8,0) resuspendiert. Nach Zugabe einer geringen Menge des rekombinanten Lysine (Phagolysin aus Listeriaphagen A118, in 20 mM Tris-HCI, pH 7,3, 20 mM NaCI, 0,1 % Triton X100) wird etwa 2-5 Minuten bei Raumtemperatur inkubiert, bis die trübe Suspension fast völlig klar geworden ist. Nach Deproteinierung der Suspension (mit Phenol:Chloroform:lsoamylalkohol, 125:24:1 (V/V), pH 4,2) kann die RNS durch ethanolische Fällung direkt gewonnen werden. Reste von DNS können gegebenenfalls durch hydrolytische Spaltung mit DNAse entfernt werden. Die nun völlig reine RNS steht für weitere Untersuchungen zur Verfügung.

### Beispiel 4: Spaltungsspezifität des Phagolysins PLY118

Rein- und Mischkulturen von verschiedenen Bakterien werden hergestellt:
a) Listeria monocytogenes
b) Listeria ivanovii
c) Staphylococcus aureus
d) Bacillus cereus
e) Escherichia coli
f) Mischkultur aus Staph. aureus, B. cereus und E. coli
g) Mischkultur aus L. monocytogenes, Staph. aureus, B. cereus und E. coli.
   Die Bakterienzellen werden anschließend gewonnen und unter Bedingungen, wie sie im wesentlichen im Beispiel 1 genannt sind, mit Lysin PLY118 lysiert. Das ATP im Lysat und auch als Kontrollmessung (ohne Zusatz von Lysin) wird durch Lumineszenzmessung bestimmt.

Alle Kontrollmessungen zeigen, daß ohne Zugabe von Lysin keine nennenswerte Zellysis gefunden wird. Weiterhin zeigt sich, daß nur Zellen der Gattung Listeria lysiert werden, wobei die Lyse unabhängig von Begleitflora sowohl in Reinkultur als auch in Mischkultur erfolgt.

## Patentansprüche

1. Gereinigtes Listeriaphagenlysin aus den Phagen A188, A500 oder A511, ausgewählt aus der Gruppe bestehend aus den Listeriaphagenlysinen PLY188, PLY500 und PLY511.

2. Phagenlysin nach Anspruch 1, **gekennzeichnet durch** eine Sequenz nach SEQ.ID.NO.:4.

3. Phagenlysin nach Anspruch 1, **gekennzeichnet durch** eine Sequenz nach SEQ.ID.NO.:5

4. Phagenlysin nach Anspruch 1, **gekennzeichnet durch** eine Sequenz nach SEQ.ID.NO.:6.

5. Nukleinsäuresequenz, codierend ein Phagenlysin nach Anspruch 1.

6. Nukleinsäuresequenz nach Anspruch 5, **gekennzeichnet durch** eine Sequenz nach SEQ.ID.NO.:1.

7. Nukleinsäuresequenz nach Anspruch 5, **gekennzeichnet durch** eine Sequenz nach SEQ.ID.NO.:2.

8. Nukleinsäuresequenz nach Anspruch 5, **gekennzeichnet durch** eine Sequencz nach SEQ.ID.NQ.:3.

## Claims

1. Purified Listeria phage lysine from phages A188, A500 or A511 chosen from the group consisting of the Listeria phage lysines PLY188, PLY500 and PLY511.

2. Phage lysine according to claim 1, **characterized by** a sequence according to SEQ.ID.NO.:4.

3. Phage lysine according to claim 1, **characterized by** a sequence according to SEQ.ID.NO.:5.

4. Phage lysine according to claim 1, **characterized by** a sequence according to SEQ.ID.NO.:6.

5. Nucleic acid sequence coding a phage lysine according to claim 1.

6. Nucleic acid sequence according to claim 5, **characterized by** a sequence according to SEQ.ID.NO.:1.

7. Nucleic acid sequence according to claim 5, **characterized by** a sequence according to SEQ.ID.NO.:2.

8. Nucleic acid sequence according to claim 5, **characterized by** a sequence according to SEQ.ID.NO.:3.

## Revendications

1. Lysine purifiée de phage de *Listeria*, provenant des phages A188, A500 ou A511, choisie dans le groupe constitué par les lysines de phage de *Listeria* PLY188, PLY500 et PLY511.

2. Lysine de phage selon la revendication 1, **caractérisée par** une séquence selon SEQ ID n° 4.

3. Lysine de phage selon la revendication 1, **caractérisée par** une séquence selon SEQ ID n° 5.

4. Lysine de phage selon la revendication 1, **caractérisée par** une séquence selon SEQ ID n° 6.

5. Séquence d'acide nucléique codant pour une lysine de phage selon la revendication 1.

6. Séquence d'acide nucléique selon la revendication 5, **caractérisée par** une séquence selon SEQ ID n° 1.

7. Séquence d'acide nucléique selon la revendication 5, **caractérisée par** une séquence selon SEQ ID n° 2.

8. Séquence d'acide nucléique selon la revendication 5, **caractérisée par** une séquence selon SEQ ID n° 3.
